(19) Europäisches Patentamt
European Patent Office
Office européen des brevets

(11) Publication number: 0 297 946
A2

(12) # EUROPEAN PATENT APPLICATION

(21) Application number: 88401523.1

(22) Date of filing: 17.06.88

(51) Int. Cl.4: A 61 K 31/40
A 61 K 31/71

(30) Priority: 19.06.87 US 63944

(43) Date of publication of application:
04.01.89 Bulletin 89/01

(84) Designated Contracting States:
AT BE CH DE ES FR GB GR IT LI LU NL SE

(71) Applicant: SYRACUSE UNIVERSITY
Syracuse New York 13244 (US)

(72) Inventor: Fondy, Thomas P.
964 Meadowbrook Drive
Syracuse, N.Y. 13224 (US)

Bousquet, Peter
32 Vernon Street
Fitchburg, Mass. 01420 (US)

Loucy, Karen J.
227, Kensington Place
Syracuse, N.Y. 13210 (US)

(74) Representative: Warcoin, Jacques et al
Cabinet Régimbeau 26, avenue Kléber
F-75116 Paris (FR)

(54) **Cytochalasin purification methods and compositions.**

(57) The isolation and purification of cytochalasins from mold cultures, specifically purification of cytochalasins A and B from Drechslera dematioidea and of cytochalasin D from mold cultures of Zygosporium masonii is described. Cytochalasins may be used in in vivo chemotherapy of invasive and metastatic disease, and in combination chemotherapy against neoplasms. Vehicles such as liposomes, which afford sustained release of the cytochalasin, may be used in the administration of such compounds.

EP 0 297 946 A2

Description

# CYTOCHALASIN PURIFICATION METHODS AND COMPOSITIONS

## BACKGROUND OF THE INVENTION

The present invention is directed to the preparation and use of synthetic and mold-derived (naturally-occurring) membrane-acting compounds, i.e., the cytochalasins, specifically cytochalasins A, B, and D. The purification of other naturally-occurring cytochalasins, and the use of these or synthetic derivatives of cytochalasins, including the related chaetoglobosins, are also embraced by this invention.

The cytochalasins, membrane- and transport-acting compounds (also having cytoskeletal-acting effects), which include the congeneric cytochalasins A-M as well as the semi-synthetic derivatives 7,20-di-0-acetyl-cytochalasin B, 7-mono-0-acetyl-cytochalasin B, and 21,22-dihydro-cytochalasin B together with the related chaetoglobosins constitute a class of more than 24 structurally and functionally related alkaloid metabolites produced by molds (Yahara et al., 1982, J. Cell Biol., 92:69-78, and Rampal et al., 1980, Biochem., 19:679-683). These substances are known to alter a wide variety of cellular functions in many different types of normal and neoplastic cells and tissues in culture (Miranda et al., 1974, J. Cell Biol., 61:481-500) and to exhibit differential effects in some cases between normal and neoplastic cell types (for references, see Lipski et al., 1987, Anal. Biochem., 161:332-340). The various congeners of these agents alter the biochemistry of fundamental cellular processes controlled by cytoskeletal and plasma membrane interactions.

Some of the cytochalasins, for example cytochalasin B (CB) pictured below, the most extensively studied of the congeners, inhibit hexose and amino acid transport in normal and neoplastic cells (Kletzien et al., 1973, J. Biol. Chem., 248:711-719, and Greene et al., 1976, Exp. Cell. Res., 103:109-117, respectively).

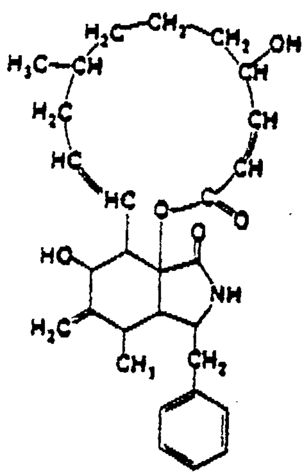

Cytochalasin B

The cytochalasins are also known to alter microfilament morphology (Schliwa, 1982, J. Cell Biol., 92:79-91), thereby affecting the cellular functions that depend upon microfilament biochemistry. Some of the cellular processes sensitive to the cytochalasins are phagocytosis, pinocytosis, cytokinesis, secretion, and exocytosis, as well as functions requiring movement and/or intercellular adherence including intracellular organelle movement and intercellular transport, cell motility, transport across tissue barriers, and a variety of immunological responses.

Phalloidin, another mold alkaloid isolated from the mushroom Amanita phalloides, blocks cell movements that require the turnover of microfilaments. It binds to actin units in microfilaments, preventing their depolymerization, effectively locking them. Thus phalloidin or analogs of phalloidin antagonize effects of cytochalasins and constitute another group of membrane-directed agents included in the present invention.

In vitro cell cultures have shown CB and other cytochalasin congeners to be non-cytotoxic at concentrations up to 100 ug/ml (200 uM) in short-term exposure of about 2 hours (Tsuruo et al., 1986, Biochem. Pharmacol., 35:1087-1090). However, prolonged exposure of the cells (in vitro) to CB results in reversible cytotoxicity. Such reversibility is observed upon removal of the CB from the cellular environment (Lipski et al., 1987, Anal. Biochem., 161:332-340). Studies on the in vitro effects of the cytochalasins on transformed (neoplastic) cells showed some efficacy. For example, O'Neill (1975, Cancer Res., 35:3111-3115), Medina et al. (1980, Cancer Res., 40:329-333), Kelly et al., (1973, Nature New Biol., 242:217-219), and Defendi

et al., 1972, Nature New Biol., 242:24-26) showed differential effects between normal and neoplastic cells, involving nuclear division and cytokinesis resulting in multinucleation of neoplastic cells. However, early in vivo chemotherapeutic studies did not reveal anti-tumor activity (Katagiri et al., 1971, J. Antibiotics, 24:722-723, and Minato et al., 1973, Chem. Pharm. Bull., 21:2268-2277). In fact, intravenous administration of B16F10 cells, treated with CB in vitro showed an increase in extra-pulmonary metastases (Hart et al., 1980, J. Natl. Cancer Inst., 64:891-900).

In spite of the dramatic effects demonstrated by the cytochalasins and related substances on cells and tissues in vitro, there have been few studies conducted with these substances in vivo. One reason for this lack of studies is the relative scarcity of the compounds of this class due to the existing inadequate isolation and purification methods. A study of any in vivo utility of the cytochalasins would require the maintenance of bioactive levels of these substances in potential target tissues. Such a study would require (1) cytochalasins in amounts that are not readily available from commercial sources; and (2) formulations of these compounds which enhance their bioavailability in vivo.

Existing preparatory techniques for the separation and purification of cytochalasins require large multiliter extractions utilizing boiling chloroform (Tanenbaum et al., in: Advances in Biotechnology, (Moo-Young, ed.), 1981, p. 423-429, Pergamon, N.Y.). Such a procedure involves the tedious process of boiling chloroform for a long period, followed by filtration and large volume solvent removal. Such a process is slow, involves large volumes of an expensive organic solvent, and is potentially hazardous. The current process is a batch adsorption technique of cytochalasins from an aqueous suspension, wherein the organic compounds are adsorbed to C18 silica gel columns. Following the adsorption step, an organic solvent extraction process isolates the cytochalasin which is purified from preparative TLC or preparative HPLC.

A second reason for the lack of in vivo study of the cytochalasins are that methods for the formulation, in vivo administration, and recovery and analysis of the cytochalasins have not yet been established. No report of in vivo administration of cytochalasin B has appeared and only limited information on cytochalasin D has been published. Also, in vivo pharmacokinetic distribution as a function of formulation and route of administration had not been carried out prior to the present disclosure.

Should extensive in vivo administration of the cytochalasins be contemplated, knowledge of their biological activity, tissue and blood distribution, and toxicity will become necessary in relation to establishing dosing schedules. This need is especially true for cytochalasins since their reversible mode of action in vitro suggests that maintenance of continuous pharmacologically active levels in vivo will be required in order to determine potentially useful effects in tumor-bearing hosts.

The maintenance of the cytochalasins in target tissues at bioactive levels can be achieved by use of delivery systems designed for the sustained release of these substances. Delivery systems that are amenable to various routes of administration, for example, subcutaneous, intraperitoneal, and intravenous routes would best be used. Such a delivery system is liposomes.

Liposomes are completely closed lipid bilayer membranes containing an entrapped aqueous volume. Liposomes may be unilamellar vesicles (possessing a single membrane bilayer) or multilameller vesicles (onion-like structures characterized by multiple membrane bilayers, each d from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "heads" orient towards the aqueous phase. This basic structure of liposomes may be made by a variety of techniques known in the art of liposomology, some of which are discussed hereinbelow.

In connection with the requirements for improved preparation and purification, in vivo study, and formulation and delivery, including liposomes, the present invention discloses a purification procedure for the cytochalasins from molds containing these compounds. Specifically, cytochalasins A and B can be purified from the mold Drechslera dematioidea and cytochalasin D from Zygosporium masonii. Other cytochalasins can be similarly purified. The present invention also discloses formulations for the in vivo administration of cytochalasin B, and methods for assaying the cytochalasins from in vivo tissues in both normal and neoplastic models. Biodistribution data of the cytochalasins in both normal and neoplastic models is presented. In vivo cytotoxicity studies of the present invention suggest the use of the cytochalasins as chemotherapeutic agents, or chemotherapeutic amplifiers in combination chemotherapy. In this regard, a further aspect of the invention is to disclose drug synergy in vitro using a combination of cytochalasins and an antineoplastic agent (specifically, doxorubicin).

## BRIEF DESCRIPTION OF THE DRAWING

FIGURE 1 shows growth of SC Madison 109 carcinoma (M109) tumors in animals treated with vehicle (CM-cellulose (1%)/Tween-20 (2%)) (closed circles) compared to animals treated SC with CB (A) at 150 mg/kg single bolus dose one day after tumor challenge or (B) at 100 mg/kg single bolus dose administered when tumors first became detectable (TAD) (open circles).

FIGURE 2 presents the effect of CB on the formation of spontaneous pulmonary metastases in animals from Figure 1(B) as determined at necropsy either at Day 28 after tumor challenge, or at the terminal stage

(days 29-36 for vehicle controls, 28-50 for CB treated). The difference between vehicle controls and treated is significant (p 0.05) Legend:: wide-spaced diagonal lines: Vehicle, at day 1 or TAD; dotted: CB at 100 mg/kg, TAD; narrow-spaced diagonal lines: S.E.M. Fractions= Number of mice with one or more nodules/total mice.

FIGURE 3 demonstrated the tissue distribution of CB administered IP bolus 50 mg/kg at 0, 3, and 12 hours post-administration. Legend:: dotted: liver; narrow-spaced left-ascending diagonals: renal fat; horizontal lines: mesentery; narrow-spaced right-ascending diagonals: pancreas; wide-spaced right-ascending diagonals: spleen; wide-spaced left-ascending diagonals: kidney.

FIGURE 4 shows the dose-response effect of CB given peri-tumorally subcutaneously (SC) in B16F10 mice, day 0. Legend: + CB 100 mg/kg; box CB 10 mg/kg; x vehicle CMC/Tw.

FIGURE 5 shows in vitro synergism by isobolo-analysis between doxorubicin (ADR) and cytochalasin B (CB) against doxorubicin-resistant P388 leukemia cells using the inhibitory concentration (IC) values after 3 hours of drug exposure. Adriamycin was added first, followed 5-15 minutes later by addition of CB. Legend: broken line = additivity line. Solid line with box = IC80.

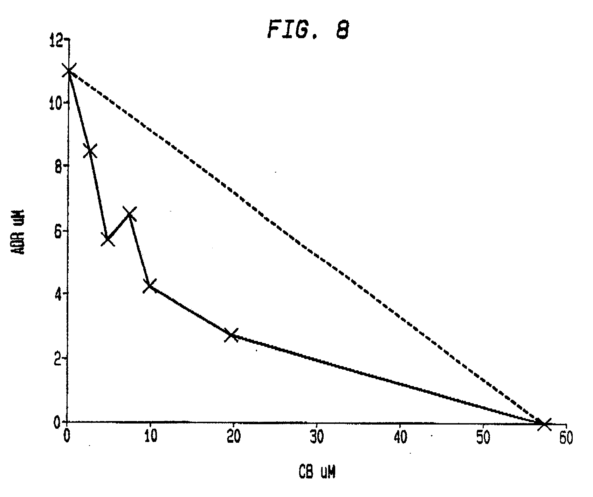

FIGURE 6 shows the IC50 isobologram when CB and doxorubicin are mixed together prior to simultaneous administration to the cells. There is strong synergism at low CB concentrations. Legend: broken line = Additivity line. Solid line with box = IC50.

FIGURE 7 shows synergism at all doses when doxorubicin is administered followed 30 minutes later by administration of CB, then complete removal of both CB and doxorubicin after 3 hours. There is strong synergism at all CB concentrations. Legend : broken line = Additivity line. Solid line with box = IC50.

## SUMMARY OF THE INVENTION

The present invention discloses a method for purifying a mold-derived membrane-acting compound comprising the step of extracting the mold matte producing the desired compound by a batch absorption technique. The mold-derived membrane-acting compound is a cytochalasin, such as cytochalasin B, D, or A, and this cytochalasin is substantially free (contains less than 1%) of other cytochalasins.

In particular, the purification method involves the steps of extracting mold matte with organic solvent, adding an aqueous solution to the filtered matte extract, separating the aqueous miscible organic solvent solution by a batch adsorption technique, extracting the resulting fractions with organic solvent, and recrystallizing the fractions on Linear K silica plates to purify the cytochalasin. The mold matte used may be D. dematioidea.

The invention also discloses pharmaceutical unit dosage forms comprising a cytochalasin and a pharmaceutically acceptable carrier or diluent, wherein the cytochalasin is cytochalasin B, and is adapted for parenteral or oral administration.

The invention also discloses a method of treating neoplasms comprising the step of administering an antineoplastic disease-treating effective amount of a pharmaceutical composition of a purified cytochalasin (such as cytochalasins A, B, or D) to a subject. Alternatively, the cytochalasin may be delivered in a liposome. These cytochalasins and liposomes containing them may also be administered with antineoplastic agents, such as doxorubicin.

Also disclosed are methods for inhibiting neoplastic cell growth in vitro comprising the step of administering synergistically-acting effective amounts of a synthetic or mold-derived membrane-acting compound and an antineoplastic agent to the cells. The synthetic or mold-derived membrane-acting compound are cytochalasins, such as cytochalasin B, and the antineoplastic agent may be doxorubicin.

When administered together, the cytochalasin B and doxorubicin can be administered to the cells simultaneously or sequentially. For example, the doxorubicin can be administered to the cells prior to the cytochalasin B, and preferably the total drug combination exposure time is 3 hours.

## DETAILED DESCRIPTION OF THE INVENTION

The following abbreviations and definitions will be employed:
ADR - doxorubicin: trademark Adria Labs, available from Aldrich Chemical Co.
CA - cytochalasin A
CB - cytochalasin B
CD - cytochalasin D
CMC, CM-Cellulose - carboxymethyl cellulose
CMC/Tw - carboxymethyl cellulose/Tween
Cytochalasins - synthetic or naturally occurring mold-derived membrane-acting compounds.
DMSO - dimethyl sulfoxide
FAT - vesicles made by a freeze and thaw technique
HPLC - high performance liquid chromatography

IC - inhibitory concentration; IC50 = 50 percent of cells inhibited
IP - intraperitoneal
IV - intravenous
LD - lethal dose
LUV - large unilamellar vesicles
LUVET - LUVs made by the VET technique
Membrane-acting compounds - naturally-occurring and synthetically derived-compounds consisting of the cytochalasins, chaetoglobosins, and phalloidins, having membrane-, transport-, and cytoskeletal (microfilament)- acting effects.
M109 - Madison 109 lung carcinoma
MLV - multilamellar vesicles
Mold-derived membrane-acting compound - naturally-occurring compounds belonging to the group consisting of the cytochalasins, chaetoglobosins, and phalloidins, having membrane-, transport-, and cytoskeletal (microfilament)- acting effects.
MTD - maximum tolerated dose
NMR - nuclear magnetic resonance
PBS - phosphate buffered saline
SC - subcutaneous
SPLV - stable plurilamellar vesicles
TAD - tumor appearance day
TLC - thin layer chromatography
Tw - Tween (polyoxyethylene sorbitan monoalkyl ethers); surface-acting agents
VET - vesicles made by an extrusion technique

The present invention discloses a new, improved process for the purification of cytochalasins, for example, CB, from mold mattes of fungi. For example, CA and CB can be isolated from mold mattes of D. dematioidea, and cytochalasin D from mold mattes of Z. masonii. The invention also discloses formulations and methods for the in vivo administration of the purified cytochalasin B, new analysis protocols for the identification of the CB in tissues, and delivery systems such as liposomes for the sustained release in vivo of the synthetic and naturally-occurring mold-derived membrane-acting compounds (cytochalasins) purified by the batch absorption methods of this invention. Cytochalasin D may also be purified from cultures of the molds Z. mycophylum and Metarrhizium anisophilae, or other mold sources known to produce CD. Cytochalasin A may also be similarly purified, or alternatively, oxidized by a standard procedure from CB. A further aspect of the invention is drug synergy between the synthetic and naturally-occuring mold-derived membrane-acting compounds, (i.e., the cytochalasins), and antineoplastic agents.

For the purification methods of the present invention, molds containing cytochalasins, for example, the mold D. dematioidea (ATCC 24346), or Z. masonii (ATCC 20011) may be obtained in dehydrated form from the American Type Culture Collection and rehydrated in an aqueous solution such as sterile distilled water. Following passaging, the mold can be maintained on agar slants at about 20°C-30°C, preferably about 25°C. Suitable agar includes 3.9% potato dextrose (Difco), or the medium defined by Tanenbaum et al. (supra.) can be used: 1% bacto-soytone (soybean enzymatic hydrolysate), dextrose, 2% yeast extract, 15% sucrose, tap water, and 20% crushed shredded wheat. Following a 12 to 14 day growth period (28 to 30 days for Z. masonii), the mold matte is harvested and freeze dried using standard freeze-drying techniques, and the resulting lyophilizate blended with an organic solvent such as methanol in an inert atmosphere such as argon or nitrogen, preferably nitrogen. Analysis of the medium used to grow the mold indicates that CB is contained within the mold matte, with no CB released into the medium.

The organic solvent extract is then rendered aqueous by addition of water, to yield about 80% aqueous solvent. The aqueous methanol suspension is then separated by a batch absorption technique, wherein an aqueous suspension is adsorbed preferably on a reverse-phase C18 silica-gel bed (Waters), and then the non-organic components removed under suction of by gravity filtration, with the cytochalasin adsorbed quantitatively to the gel bed. About 10 to 20 liters, preferably 15 liters of extract may be passed through about 45 g of silica-gel. This corresponds to about 1800 g wet weight of mold. The filter pack is dried in vacuo, then washed with about 500 ml of organic hydrocarbon, for example, hexane, then with a 1:1 (v/v) mixture of solvents such as tetrahydrofuran:hexane. Alternatively, a linear gradient of tetrahydrofuran:hexane may also be used to purify the cytochalasin. In choosing the solvents to be used in this step, any organic solvent of minimum polarity to elute CB, without eluting contaminating organics, may be employed.

Early fractions of crude CB crystallize spontaneously, and may be combined with later fractions and rotary evaporated at 30 to 40°C. The resulting film may be recrystallized at about 50°C according to standard techniques, from an organic solvent such as chloroform or an organic solvent pair such as preferably chloroform:hexane (1:1 v/v). Resulting crystalline material can then be further purified by preparative TLC on silica gel plates (preferably Linear K plates with a sample concentration zone) according to standard techniques, and the zones containing CB and CA recovered with methanol and recrystallized according to standard techniques, from organic solvent, such as toluene:methanol (85:15). Additional CB from mother liquors may be recovered from normal phase silica gel column chromatography by elution with organic solvents such as 85:15 v/v toluene:methanol. Fractions containing CB and CA may be identified by TLC, evaporated to dryness, and recrystallized as above. CB may then be formulated into pharmaceutical

preparations. If desired, CA purified by this method can also be employed in vivo or in vitro. An alternative method for purifying CA from the mold is to purify CB and oxidize it according to standard oxidation methods, i.e., a gentle manganese dioxide oxidation, well known to those in the art.

Alternatively, the final purification technique may be performed using a preparative HPLC technique with standard phase silica and hexane:tetrahydrofuran (60:40), or reverse phase silica and methanol:water (70:30).

Following purification of cytochalasins by the above technique, proton NMR, HPLC, and analytical TLC performed on the isolated product demonstrated at least about 99% purity. Such purity is greater than that of cytochalasins currently available. Crystalline CB, purified by these methods had a melting point of 215° - 217°C, and showed no evidence of fluorescent-quenching, iodine vapor positive contaminants, or p-anisaldehyde positive contaminants in two solvent systems when 100 ug was analyzed by TLC according to standard techniques. HPLC analysis (performed according to standard procedure) produced a single, non-contaminated peak. NMR spectra of CB, obtained according to standard techniques, coincided exactly with that of published spectra of CB, without contamination by CA. It is an embodiment of this invention to provide purified naturally-occurring cytochalasins substantially free (containing less than about 1%) of contaminating cytochalasins. For example, it is an object of the invention to provide purified naturally-occurring cytochalasin B substantially free of contaminating cytochalasins, such as cytochalasin A.

A further aspect of the invention involves the in vivo administration, and recovery and analysis of the cytochalasins from tissues. To determine protocols for recovery of exogenously added CB to tissues, CB was added to excised Balb/c mouse liver, heart, pancreas, spleen, and lung by addition of 1 or 5 ug of CB in methanol to the organ and the organ chilled. The analysis of the CB in the tissues was performed according to Example 13. Almost total recovery of CB from tissues is achievable using this technique. Results of this recovery are presented in Table 1. A comparison of Cb extraction immediately following CB addition to tissues, with CB extraction 30 minutes following CB addition showed no difference in recoverable CB. This indicated the absence of CB metabolism in the chilled excised tissues.

The maximum tolerated single bolus dose (MTD) of CB ($LD_{5-10}$) was estimated as a function of vehicle and route of administration, in M109 tumor-bearing, B16F10 tumor-bearing, and normal mice (Table 2). Both $CD2F_1$ and $B6D2F_1$ mice were tested. Slightly greater sensitivity to CB was demonstrated by $CD2F_1$ as compared to $B6D2F_1$ mice. In tissue distribution studies of CB in vivo, 50 mg/kg (1000 ug/20 g mouse) was injected intraperitoneally and the distribution determined at time intervals thereafter as explained in Example 13. Following single bolus injection, CB distributes itself in mesentery, renal fat, kidneys, and blood, and appears at the highest levels in liver, spleen, and pancreas (Figure 3). It is cleared from the spleen and pancreas by 12 hours, but persists in liver for 72 hours. Some CB is metabolized within the first few minutes after injection, but the remaining 35% persists in liver for up to 72 hours. Sustained release formulations such as liposomes would maintain active CB in tissues.

In studies demonstrating dose-limiting host toxicity of CB administered to Balb/c, $B6D2F_1$, or $CD2F_1$ mice with the M109 lung carcinoma, various routes of administration were tested for a single bolus drug in a variety of formulations. The vehicles used in the invention may be selected with regard to the intended route of administration and standard pharmaceutical practice. The preparations can be injected parenterally, for example, intra-arterially or intravenously. The preparations may also be administered via oral, subcutaneous, intraperitoneal, or intramuscular routes. For parenteral administration, they can be used, for example, in the form of a sterile pyrogen-free aqueous solution which may contain other solutes.

For example, in the present studies, 0.85% (wt/v) sterile sodium chloride solution, sterile pyrogen-free CM-cellulose (1%)Tween-20 (2%), 10% Intralipid (Cutter Laboratories), or 48% ethanol/sodium chloride (v/v) solution were used as vehicles. Liposomes can alternatively be used as a vehicle. In such cases, the CB can be entrapped by any of the methods known in the art for forming liposomes, mentioned hereinbelow, and administered by any of the above-named routes of administration.

The MTD of CB suspension in pyrogen-free carboxymethyl cellulose(1%)/Tween -20 (2%) was 100 mg/kg SC, 50 mg/kg IP, and 10 mg/kg IV when administered to $CD2F_1$ mice. In DMSO, the MTD was 25 mg/kg IP, and less than 100 mg/kg SC, and in 33% ethanol, 10 mg/kg IV. At doses of 100 -150 mg/kg CB administered SC in CMC/Tw to $CD2F_1$ mice that had sustained challenge 24 hr with M109 tumor cells implanted SC, appearance of measurable tumor nodules were delayed by 66%.

For SC tumors, growth was monitored by calculating tumor weight according to the methods of Johnson et al. (1975, Cancer Chemo. Rep., 59(4):697-705). For IP and IV-induced tumors, growth was determined by host survival. Metastases were determined at necropsy by fixation of the organ in Bouins solution according to standard procedure, and metastatic foci counted under a dissecting microscope (see Table 1).

In addition to displaying anti-tumor effects when administered alone, the present invention discloses that cytochalasins act synergistically with at least one antineoplastic agent in vitro. Specifically, in the case of CB, simultaneous or sequential administration with doxorubicin (ADR) results in inhibition of neoplastic cell growth and viability in vitro. In particular, P388 cells cultured in micro-titer plates (24- or 96-well plates) were used to determine drug effects by colony formation in soft agar immediately following drug exposure. Viability was ascertained by dye reduction tests and/or Coulter counting of numbers of regrown cells.

Following culture in microtiter plates, the cells were exposed to various dilutions of ADR and CB according to a standard microbioligical checkerboard technique (Lorian, M., ed., Antibiotics in Laboratory Medicine, 2nd ed., Williams & Wilkins, Baltimore). Standard exponential dilutions (see Example 15) were interpreted by the pattern they form (determined by the presence of cell growth and viability following treatment with the drug

combination) when plotted on the arithmetic scale isobologram. Depending on resultant cell growth and the reaction of the cells to the two drugs, the isobologram depicts (1) a merely additive effect of the two drugs, (2) an antagonistic drug effect, resulting in lower inhibition of cell growth than that observed with either drug alone, or (3) reduction in growth and viability, in excess of what would be expected with either drug alone. Drug combinations are considered to be synergistic if inhibition occurs at concentrations which lie below the additivity effect line (the line described by the inhibitory concentration of choice of each drug alone, for example, the IC50, IC90, or IC100).

ICs for cytochalasins and antineoplastic agents were determined by inoculating wells containing cultured cells with dilutions of the desired compound, and the resulting exposed cell viability determined at a fixed timepoint after exposure (i.e., 20 or 48 hours). The concentration of the compound that is found to inhibit 50% of the initial cells cultured is the IC50 of that compound with respect to those cells. The IC90 and IC100s can similarly be determined.

To determine drug synergy, following seeding of microtiter wells with neoplastic cells, triplicate wells were treated with mixtures of CB and ADR (dilutions determined by the results of the IC determination experiments as previously described) either simultaneously or sequentially (ADR administered first, with CB administered either about 5-15 minutes or 30 minutes later). After 3 hours incubation, the plates were centrifuged, media removed, cells washed and refed and plated out in 96- well plates and incubated for 48-72 hours.

The antineoplastic agents which can be used in the practice of the present invention include but are not limited to the anthracyclines such as doxorubicin, daunorubicin, or epirubicin, the vinca alkaloids such as vinblastine or vincristine, the purine or pyrimidine derivatives such as 5-fluorouracil, the alkylating agents such as mitoxanthrone, mechlorethamine hydrochloride, or cyclophosphamide, the platinum compounds such as cis-platinum, the folic acid analogs such as methotrexate, and the antineoplastic antibiotics such as mitomycin or bleomycin.

As stated hereinabove, liposomes may be used as a sustained release delivery vehicle for the administration of the cytochalasins and/or the antineoplastic agent (i.e., ADR) of the present invention. Any of the techniques known in the art for making liposomes may be used in this invention. For example, the original liposome preparation of Bangham et al. (J. Mol. Biol., 1965, 12:238-252) involves suspending phospholipids in an organic solvent which is then evaporated to dryness leaving a phospholipid film on the reaction vessel. Next, an appropriate amount of aqueous phase is added, the mixture is allowed to "swell," and the resulting liposomes which consist of multilamellar vesicles (MLVs) are dispersed by mechanical means. This technique provides the basis for the development of the small sonicated unilamellar vesicles described by Papahadjopoulos et al. (Biochim. Biophys, Acta., 1968, 135:624-638), and large unilamellar vesicles.

Unilamellar vesicles may be produced using an extrusion apparatus by a method described in Cullis et al., PCT Publication No. WO 87/00238, published January 16, 1986, entitled "Extrusion Technique for Producing Unilamellar Vesicles" incorporated herein by reference. Vesicles made by this technique, called LUVETS, are extruded under pressure through a membrane filter. Vesicles may also be made by an extrusion technique through a 200 nm filter; such vesicles are known as $VET_{200}$s.

Another class of liposomes that may be used are those characterized as having substantially equal lamellar solute distribution. This class of liposomes is denominated as stable plurilamellar vesicles (SPLV) as defined in U.S. Patent No. 4,522,803 to Lenk, et al., monophasic vesicles as described in U.S. Patent No. 4,558,579 to Fountain, et al. and frozen and thawed multilamellar vesicles (FATMLV) wherein the vesicles are exposed to at least one freeze and thaw cycle; this procedure is described in Bally et al., PCT Publication No 87/00043, January 15, 1987, entitled "Multilamellar Liposomes Having Improved Trapping Efficiencies" and these references are incorporated herein by reference.

Any of the methods for making liposomes can be used to encapsulate the synthetic or naturally occurring mold-derived membrane-acting compounds (i.e., the cytochalasins) purified by the methods of this invention. Pharmaceutical compositions of these liposomal forms of the cytochalasins can be administered in vitro or in vivo as described hereinbelow.

A variety of sterols and their water soluble derivatives have been used to form liposomes; see specifically Janoff et al., U.S. Patent No. 4,721,612, issued January 26, 1988 entitled "Steroidal Liposomes." Mayhew et al. (PCT Publication No. WO 85/00968, published March 14, 1985) described a method for reducing the toxicity of drugs by encapsulating them in liposomes comprising alpha-tocopherol and certain derivatives thereof. Also, a variety of tocopherols and their water soluble derivatives have been used to form liposomes, see Janoff et al., PCT Publication No. 87/02210, April 23, 1987, entitled "Alpha Tocopherol-Based Vesicles."

Alternatively, when an ionizable antineoplastic agent such as doxorubicin is employed, the liposomes can be loaded with drug according to the procedures of Bally et al., PCT Publication No. 86/01102, published February 27, 1986, and incorporated herein by reference. This technique allows the loading of antineoplastic agents by creation of a transmembrane concentration gradient across the liposome membranes. This gradient is generated by a concentration gradient for one or more ionic species (e.g., $Na^+$, $Cl^-$, $K^+$, $Li^+$, or $H^+$) across the liposome membranes. Preferably, these ionic gradients are pH ($H^+$) gradients, which drive the uptake of the ionizable antineoplastic agent across the liposome membranes. Once the liposomes are loaded with the antineoplastic agent(s) by this or any other method, pharmaceutical formulations can be made which can be delivered in vitro or in vivo as described hereinbelow.

In a liposome-drug delivery system, a bioactive agent such as a drug is entrapped in or associated with the liposome and then administered to the patient to be treated. For example, see Rahman et al., U.S. Patent

No. 3,993,754; Sears, U.S. Patent No. 4,145,410; Papahadjopoulos et al., U.S. Patent No. 4,235,871; Schneider, U.S. Patent No. 4,114,179; Lenk et al., U.S. Patent No. 4,522,803; and Fountain et al., U.S. Patent No. 4,588,578. In the present invention, as mentioned hereinabove, the synthetic and naturally-occuring mold-derived membrane-acting compounds purified by the methods of this invention (i.e., cytochalasins, derivatives thereof, or chaetoglobosins) may be entrapped in or associated with liposomes. Alternatively or additionally, antineoplastic agents as described hereinabove can be co-entrapped in liposomes, or entrapped in liposomes co-administered with those liposomes containing the cytochalasins. Such formulations of liposomes may be administered simultaneously or sequentially.

During preparation of the liposomes, organic solvents may be used to suspend the lipids. Suitable organic solvents are those with a variety of polarities and dielectric properties, which solubilize the lipids, and include but are not limited to chloroform, methanol, ethanol, dimethylsulfoxide (DMSO), methylene choloride, and solvent mixtures such as benzene:methanol (70:30). As a result, solutions (mixtures in which the lipids and other components are uniformly distributed throughout) containing the lipids are formed. Solvents are generally chosen on the basis of their biocompatability, low toxicity, and solubilization abilities.

The liposomes of the present invention containing the cytochalasins and/or antineoplastic agents, and the pharmaceutical formulations thereof can be used therapeutically in animals (including man) in the treatment of infections or conditions which require repeated administrations or the sustained delivery of the compounds in their active forms. Such conditions include but are not limited to neoplasms such as those that can be treated with cytochalasins, synthetic or naturally-occuring, which are purified by the methods of this invention, or antineoplastic agents and cytochalasins.

Other drug delivery systems can also be used to administer the synthetic and naturally occurring mold-derived membrane-acting compounds purified by the present invention. Such delivery systems include but are not limited to osmotic pumps, transdermal patches, infusion pumps, osmotic pumps, biodegradable polymers, monoclonal antibody-linked systems, suppositories, rhinile, dragees, troches, and the like.

The mode of administration of (1) the cytochalasins, synthetic or naturally occurring which have been purified by the methods of the present invention, or (2) the cytochalasins as described in (1) with antineoplastic agents, or (3) the cytochalasins and antineoplastic agents delivered in liposomes, and the pharmaceutical formulations thereof, may determine the sites and cells in the organism to which the compound will be delivered. The formulations, including the liposomes of this invention can be administered alone but will generally be administered in admixture with a pharmaceutical carrier selected with regard to the intended route of administration and standard pharmaceutical practice. The compositions can be provided in pharmaceutical unit dosage forms. Such pharmaceutical dosage forms can be adapted for oral and parenteral administration. The preparations can be injected parenterally, for example, intravenously. For parenteral administration, they can be used, for example, in the form of a sterile, pyrogen-free aqueous solution which may contain other solutes, for example, enough salts or glucose to make the solution isotonic. The formulations may also be administered subcutaneously for example at the site of metastases. Other uses, depending upon the particular properties of the preparation, may be envisioned by those skilled in the art.

For the oral mode of administration, the formulations of this invention can be used in the form of tablets, capsules, losenges, trochees, powders, syrups, elixirs, aqueous solutions and suspensions, and the like. In the case of tablets, carriers which can be used include lactose, sodium citrate and salts of phosphoric acid. Various disintegrants such as starch, and lubricating agents, such as magnesium stearate, sodium lauryl sulfate and talc, are commonly used in tablets. For oral administration in capsule form, useful diluents are lactose and high molecular weight polyethylene glycols. When aqueous suspensions are required for oral use, the active ingredient is combined with emulsifying and suspending agents. If desired, certain sweetening and/or flavoring agents can be added.

It is to be understood that treatment of a disease (i.e., treating a metastatic disease in vivo) using the pharmaceutical composition of the invention is also to include the curative, remissive, retardive, or prophylactic treatment using such compositions. For the administration to humans in the curative, remissive, retardive, or prophylactic treatment of neoplastic diseases the prescribing physician will ultimately determine the appropriate dosage of the antineoplastic disease-treating effective amount of the neoplastic drug for a given human subject, and this can be expected to vary according to the age, weight, and response of the individual as well as the nature and severity of the patient's disease. The dosage of the cytochalasins would be expected to be about that of one tenth the dosage producing an $LD_{10}$ in mice (one tenth the MTD) or above, however, dosages would be determined according to the specific cytochalasin used and the circumstances of treatment, and would be determined by the administering physician. The dosage of the drug in liposomal form will generally be about that employed for the free drug. In some cases, however, it may be necessary to administer dosages outside these limits.

The following examples are given for purposes of illustration only and not by way of limitation on the scope of the invention.

## EXAMPLE 1

The mold D. dematioidea (ATCC 24346), (about 25 mg) was obtained in dehydrated form from the American

Type Culture Collection and was rehydrated in sterile distilled water, forming a suspension. The mold was then passaged once and maintained on 3.9% potato dextrose agar (Difco) slants at 25°C. For preparation of cultures for cytochalasin B purification, the medium defined by Tanenbaum et al. (supra.) was used: briefly, 2 liters of medium was made using 1% bacto-soytone, 4% dextrose, 2% yeast extract, 15% sucrose, tap water, and 20% crushed shredded wheat. The medium was poured into 4 pyrex plates and autoclaved at 270°C for 90 minutes. Cooled plates were seeded with 15ml of distilled water suspension of mold and allowed to grow at ambient temperature for 14 days.

EXAMPLE 2

The procedures of Example 1 were followed to culture Zygosporium masonii, (ATCC Number 20011) which was cultured for 28 days.

EXAMPLE 3

The D. dematioidea mold matte (1400 g wet weight) grown according to the procedure of Example 1 was harvested and lyophilized using a Thermovac lyophilizer to yield 419 g of dried mold. The dried mold was then blended using a Waring blender for several minutes with 1600 ml of methanol in a nitrogen atmosphere, followed by a second extraction using 1000 ml methanol. The resulting extract was then separated from the solvent by suction filtration, and the residue was again extracted with with 800 ml methanol. Distilled water was then added to give 80% aqueous methanol solution. The aqueous methanol solution was then separated by a batch adsorption method using reverse-phase C18 silica-gel column (Waters), under suction.

15 liters of extract were passed through 45 g of silica-gel corresponding to 1800 g wet weight of mold. The bed was dried overnight in vacuo in a dessicator. The filter pack was then washed with 500 ml of hexane, then with a linear gradient of (finally) a 1:1 (v/v) mixture of tetrahydrofuran:hexane. Early fractions of crude CB crystallized spontaneously, and were mixed with later fractions and evaporated at 25°C. The resulting film was recrystallized according to standard techniques, from chloroform:hexane. Resulting crystalline material was then repurified by preparative TLC on silica gel (Linear K plates) according to standard techniques, and the zones containing CB and CA recovered, eluted with ethanol, and recrystallized from chloroform:hexane. Additional CB from mother liquors was recovered from normal phase silica gel column chromatography by elution with toluene:methanol (85:15). Fractions containing CB and CA were identified by TLC, evaporated to dryness, and recrystallized as above.

EXAMPLE 4

Madison 109 (M109) carcinoma was maintained in Balb/c and $CD2F_1$ mice by serial passage of 20% tumor brei (approximately $1X10^6$ total cells) in mice every 12 days according to the techniques of Rose (1981, Cancer Treat. Rep., 65:299-312). The $CD2F_1$ were implanted with 0.2 ml M109 SC ventrally on the abdomen. Tumor brei were prepared by excising the tumor from the passage mouse, removing any necrotic areas, weighing the tumor, mincing it, and suspending the fragments in 0.1 ml 20% (v/v) penicillin/streptomycin (Gibco) and 0.85% (w/v) sterile sodium chloride solution (total 1.0 ml/g tumor), and dispersed by repeated aspiration and expulsion through a syringe. The suspension was then diluted to 20% with 0.85% sterile sodium chloride and 0.2 ml was injected SC. Viability of the tumor suspension was about 20%, as determined by the standard trypan blue exclusion test.

The above procedure was repeated, administering the M109 tumor brei IV 90.1 ml) through the tail vein, or IP (0.2 ml).

EXAMPLE 5

Mice (6) were subcutaneously implanted with $1X10^5$ tumor cells as isolated in Example 4 on Day 0, and drug treatment was given on Day 1. CB (100 mg/kg mouse, or 0.2 ml of 10.0 mg/ml CB, per 20 gm mouse), suspended in CMC/Tw was administered to mice. See Table 2 for results of dosing.

Dose response studies were repeated using CB suspended in 0.85% sodium chloride solution, in Intralipid (Cutter Labs), and in sterile pyrogen-free CM-cellulose (1%)/Tween-20 (2%), or dissolved in DMSO. The CM-cellulose/Tween 20 was the most reliable vehicle for suspension and administration of the CB, as it delivered 80-90% of the nominal dose as determined by TLC analysis of residual CB in the syringe.

When mice which had previously an IV tumor challenge were administered 50 mg/kg CB by the IP route, no

cytotoxic effects were observed (no effect on life span). When administered SC as a single bolus dose administered either 24 hours after tumor challenge or when the tumor appeared (about day 6), however, CB was able to delay the appearance of measurable tumor nodules, to slow growth rate of the nodule to inhibit invasion, and to extend life span (Figure 1).

As shown in Figure 1, CB treatment produced a significant decrease in tumor growth rate when animals were treated SC peri-tumorally under the implant with CB either on Day 1 (Figure 1A), or when a measurable tumor nodule was observed (ie.: around Day 6) (Figure 1B). In animals treated with CB on Day 1 or when the nodule was detected, increased median survival time was observed, and 33% of the animals were tumor-free by Day 50. Lower tumor weight was detected in all the treated groups as compared to controls, although this difference lessened with advancing stage of tumor growth. As shown in .Figure 2, animals treated subcutaneously with 100 mg/kg CB at the time of tumor detection showed fewer lung tumor nodules (metastases) than control groups treated with vehicle alone. No effect on reducing tumor metastases in the liver or spleen was detected with SC administration of CB.

## EXAMPLE 6

Mice were intraperitoneally implanted with $1X10^5$ tumor cells as isolated in Example 3 on Day 0, and and drug treatment was given on Day 1. CB (0.2 ml of a 5 mg/ml solution was administered (1.0 mg CB per 20 g mouse; or 50 mg/kg) in 0.85% sodium chloride solution. See Table 2 for results of dosing.

## EXAMPLE 7

Mice were intravenously implanted with $1X10^5$ tumor cells as isolated in Example 3 on Day 0. and and drug treatment was given on Day 1. CB (0.2 mg, in 0.1 ml of 33% EtOH in 0.85% sodium chloride solution) was administered. See Table 2 for results of dosing.

Dose response studies against B16F10 tumor in mice show that CB administered subcutaneously has a measurable effect on tumor nodule growth rate (Figure 4). CB (100 mg/kg) administered peri-tumorally delays the tumor appears from Day 7 to Day 18, when treatment is administered on Day 1.

## EXAMPLE 8

The maximum tolerated dose (MTD) of CB (50 mg/kg CB) by intraperitoneal administration was determined by the following procedure. A solution of CB as isolated by Example 2 was obtained by sonication of the CB in DMSO (to make a 25 mg/kg solution of CB) for 2 minutes in an ultrasonic bath at 80-90 Hz power. The suspension (0.1 ml) was administered IP in a single bolus injection and results for its toxicity are shown in Table 2.

The above procedure was repeated for CB in CM-cellulose/Tween 20 (CMC/Tw) vehicle (0.1 ml administered).

The above procedures were repeated for 25 and 100 mg/kg CB in DMSO, and for 100 mg/kg CB in CMC/Tw.

CB administered in DMSO was approximately twice as toxic as when it was delivered in the CM-Cellulose/Tween 20 (Table 2).

## EXAMPLE 9

The maximum tolerated dose (MTD) of CB (150 mg/kg, in CMC/Tw) by subcutaneous administration over the peritoneal wall was determined by the procedure of Example 8.

CB administered in DMSO was approximately twice as toxic as when it was delivered in the CM-Cellulose/Tween 20 (Table 2).

Table 2 shows results for this procedure.

## EXAMPLE 10

The maximum tolerated dose (MTD) of CB (5 mg/kg) by intravenous administration was determined by the procedure of Example 7. The CB was suspended in 0.1 ml 95% ethanol/0.85% NaCl (1:2 v/v), heated to 37° C and administered via tail vein within 5 minutes of mixing.

The above procedure was repeated using 0.1 ml CM-Cellulose/Tween 20.

The above procedure was repeated using 10, 20, 40, and 50 mg/kg dose of CB with the CM-Cellulose/Tween 20 vehicle, and 10, 20, and 40 mg/kg CB with EtOH/NaCl.

The results for the above are shown in Table 2.

## EXAMPLE 11

The CB (2.0 mg) as purified in Example 1 is suspended in 2.0 ml of chloroform also containing 200 mg of egg phosphatidylcholine. The solution is pipetted into a round bottom flask and dried to a thin film. The film is then resuspended in 5.0 ml of PBS. The suspension is allowed to sit undisturbed for one hour, after which time it is extruded 5 times through a 5.0 um polycarbonate (straight through path) filter according to the LUVET technique, forming a large unilamellar vesicles.

The above method is repeated using 200 mg total of a 70:30 mixture of egg phosphatidylcholine : dimyristoylphosphatidylglycerol (EPC:DMPG).

## EXAMPLE 12

SPLVs containing CB are formed by drying 200 mg of egg phosphatidylcholine to a thin film from chloroform on the sides of a round bottom flask, and resuspending the film in 5.0 ml of ethyl ether, to which is added 5.0 mg of CB as purified in Example 1. The organic solvent is then removed by rotoevaporation in a water bath at 37°C, and the remaining paste is further hydrated using 10.0 ml of PBS.

## EXAMPLE 13

Livers of mice treated according to Examples 4, 5 and 6 were homogenized with a Brinkman polytron using 5.0 ml of methanol /g tissue. The homogenate was centrifuged at 5000g for 5 minutes. The supernatant was brought to 80% water using HPLC grade water and passed through Sep-Pak C-18 cartridges that had first been conditioned with 5.0 ml methanol, followed by 10.0 ml of distilled water. The cartridges were dried under vacuum and washed with 3.0 ml hexane (HPLC grade), and the CB recovered with 2.0 ml of EtOAc (HPLC grade). The elutes were evaporated under a stream of nitrogen and dissolved in 250 ul tetrohydrofuran (HPLC grade). Samples were filtered through a 0.45 um nylon syringe filter into a 50 ul sample loop. The samples were analyzed by HPLC, using a regular phase silica column, a solvent system of hexane:tetrahydrofuran (60:40), and analyzed at 230 nm. The lower limit of sensitivity was determined to be 1 ug per total extract since 20% was analyzed with a quantitative limit of 0.2 ug.

The above procedure was repeated using livers from Examples 6 and 7.

## EXAMPLE 14

Inhibitory concentrations ("ICs") of ADR, CA, and CB on P388 murine leukemia cells (drug-resistant line) were determined as follows.

Cells (5 X $10^4$) were plated out in 200 uL of RPMI 1640 medium containing 10% calf serum. CB, CA, and ADR were added to wells as follows to determine inhibitory concentration as follows: 8 wells were inoculated with dilutions of CB: 0.00 uM CB, 1.00 uM CB, 2.00 uM CB, 4.00 uM CB, 10.00 uM CB, 20.00 uM, 40.00 uM CB, and 80.00 uM CB.

Seven separate wells were inoculated with CA as follows: 1.00 uM, 2.00 uM, 4.00 uM, 10.00 uM, 20.00 uM, 40.00 uM, and 80.00 uM.

Eight wells were inoculated with ADR as follows; 1.00 nM, 2.00 nM, 4.00 nM, 10.00 nM, 20.00 nM, 40.00 nM, 100.00 nM, and 200.00 nM.

All wells were incubated at 37°C, in 5% $CO_2$ for 3 hours, after which time the plate was centrifuged at 600 X g for 5 minutes, the drug medium removed and replaced with 200 uL of fresh RPMI 1640 medium, containing 10% calf serum, centrifuged again at 600 X g, and cells refed and incubated for 45 hours at 37°C, in 5% $CO_2$. After the 45 hour incubation, the cells were analyzed for viability using the MTT reduction assay as described in Alley et al.

Results of this IC assay show that the IC50 of CB was 35-40 uM, while the IC50 of ADR was 3.5 nM.

EXAMPLE 15

P388 murine leukemia cells (P388/ADR) selected for multi-drug resistance to doxorubicin and other natural product anti-tumor agents, provided by the National Institutes of Health, were seeded at 5 X $10^4$ cells/ml in 24-well microtiter plates, 2.0 ml per well, in RPMI 1640 medium with 10% fetal calf serum and 10 uM mercaptoethanol. The cells were allowed to equilibrate and divide overnight at 37°C, in 5% $CO_2$, then treated in duplicate wells with ADR at 2.5, 5, 10, and 20 uM. Other duplicate wells were treated with CB at 2.5, 5, 7.5, 10, and 20 uM. Duplicate wells also contained the above amounts of ADR and CB, which were added to the cells simultaneously. (IC50s for ADR were 3.5 uM and 12 uM, dependent on lot of ADR; IC50s for CB were 35-40 uM). After 3 hours of incubation at 37°C, the cells from each well were transferred to sterile centrifuge tubes, and the wells washed with 2.0 ml of sterile medium to assure full cell transfer. The tubes were centrifuged at 600 x g for 5 minutes, and the drug-medium decanted and discarded. The cells were then resuspended in 2.0 ml of fresh medium and plated into 24-well plates, incubated at 37° C for 96 hours, and cell viability and growth after treatment determined by Coulter counting.

EXAMPLE 16

The materials and procedures of Example 14 were employed using 96-well microtiter plates with an inoculum of 200 ul medium and 1 X $10^4$ cells per well. After cell seeding and doubling overnight, ADR was added to thirty of the wells in dilutions as follows: 0, 50, 100, 200, and 400 nM ADR, repeated five times. Thirty minutes later, CB was added to the wells in the following dilutions: wells 1-5 were inoculated with 0 uM CB, wells 6-10 were inoculated with 10.00 uM CB, wells 11-15 with 20.00 uM CB, wells 16-20 with 40.00 uM CB, wells 21-25 with 80.00 uM CB, and wells 26-30 with 160.00 uM CB. The plates were incubated as in Example 14, and 3 hours after addition of the ADR, the medium was replaced with fresh medium. Forty eight hours later, after incubation at 37°C at 5% $CO_2$, the cells were observed for viability as described by the MTT dye reduction assay, according to the technique of Alley M., et al. (1986, Cancer Res., 48:589-601), by reading dye reduction at 540 nm.

Results of the synergy are graphically depicted in Figure 6. At high CB dose (above 10 uM CB), antagonsim was observed when CB and ADR were added simultaneously, but below 10 uM CB, strong synergy results. However, in all cases where ADR was administered first, synergy was observed at all doses.

Growth of cells was plotted on an isobologram (see Figures 5, 6, and 7), using the IC 50, IC 80 and IC 90 values as standards. Points falling below the additivity line are points depicting CB/ADR synergy. For the IC50 plots, (Figure 6), ADR doses of 7.0 uM and less, when combined with CB doses of 90 uM and less result in synergistic effects. For the IC 80 plot, (Figure 5), ADR doses of 30 uM and less, when combined with CB doses of 60 uM and less result in synergistic effects. The most potent dose combinations are those combined doses having an effect which employ the least ADR and the most CB.

EXAMPLE 17

The procedures and materials of Example 16 were repeated employing CA instead of CB. The wells contained dilutions of ADR as described in Example 16, and the dilutions of CA were as follows: wells 1-5: 0.00 uM CA; wells 6-10:2.00 uM CA; wells 11-15: 4.00 uM CA; wells 16-20: 10.00 uM CA; wells 21-25:20 uM CA; and wells 26-30: 40.00 uM CA.

The procedure for determining cell viability were as described in Example 16.

EXAMPLE 18

The materials and procedures of Example 16 were employed to determine ADR-CB synergy at an IC80 of ADR. Serial dilutions of ADR and CB were made according to Example 16, and plated out into 96-well plates. Where combined amounts of CB and ADR were inoculated, ADR was inoculated into microtiter wells followed by the addition of CB to those wells 5-15 minutes later. Dilutions of ADR were mixed with dilutions of CB and plotted on an isobologram. Drug synergy was observed at all dilutions between 30 uM and less ADR and 60 uM and less CB (Figure 5).

EXAMPLE 19

The materials and procedures of Example 11 were employed, entrapping CA in liposomes.

TABLE 1

RECOVERY OF CB AFTER EXOGENOUS ADDITION TO MOUSE TISSUES

| | UG CB ADDED | | | UG $[^3H]$-CB ADDED |
|---|---|---|---|---|
| | 1000 | 5[b] | 1 | 50[c] |
| TISSUE (WT IN MG) | % CB RECOVERED BY HPLC (+/- S.D. or RANGE) | | | % $^3H$-LABEL RECOVERED |
| LIVER (500) | 103 | 82 (10) | ND[a] | 71 (70-72) |
| HEART (140) | 88 | ND | ND | ND |
| PANCREAS (140) | ND | 94 (10) | 110 | 77 (75-79) |
| SPLEEN (85) | ND | 99 (12) | 98[c] (90-105) | 89 (87-90) |
| LUNGS (180) | ND | 94 (7) | 70 | 80 (77-82) |

a Not Determined
b 4-6 animals per group
c 2 animals per group

TABLE 2

CYTOCHALASIN B IN VIVO
MAXIMUM TOLERATED DOSES#: IP, SC, IV

| ROUTE | VEHICLE | FORMULATION | SCHEDULE (DAYS) | MG/KG | MAXIMUM WT LOSS (%) | DRUG DEATHS |
|---|---|---|---|---|---|---|
| IP | CMC/TWEEN | SUSPENSION | 1 | 50 | 7 | 2/34 |
| | CMC/TWEEN | SUSPENSION | 1,4,7 | 50/DAY | 6 | 0/16 |
| | DMSO | SOLUTION | 1 | 25 | 5 | 1/5 |
| SC | CMC/TWEEN | SUSPENSION | 1 | 100 | 3 | 0/31 |
| | CMC/TWEEN | SUSPENSION | 1 | 150 | 7 | 4/28 |
| | DMSO | SOLUTION | 1 | 100 | 7 | 5/32 |
| IV | CMC/TWEEN | SUSPENSION | 1 | 20 | 1 | 0/5 (BDF) |
| | ETOH/SALINE | SOLUTION | 1 | 20 | 1 | 0/7 (BDF) |
| | ETOH/SALINE | SOLUTION | 1 | 10 | 2 | 1/5 (CDF) |
| | ETOH/SALINE | SOLUTION | 1 | 5 | 0 | 0/29(CDF) |

# IN B6D2F1, CD2F1, OR C57B1/6 MICE

## Claims

1 A pharmaceutical unit dosage form comprising a cytochalasin and a pharmaceutically acceptable carrier or diluent.

2. The pharmaceutical unit dosage form of claim 1 wherein the cytochalasin is cytochalasin B.

3. The pharmaceutical unit dosage form of claim 2 adapted for parenteral administration.

4. The pharmaceutical unit dosage form of claim 2 adapted for oral administration.

5. A composition comprising a cytochalasin and a liposome.

6. A pharmaceutical composition comprising the composition of claim 5 and a pharmaceutically acceptable carrier or diluent.

7. The composition of claim 5 additionally comprising an antineoplastic agent.

8. The composition of claim 7 wherein the antineoplastic agent is doxorubicin.

9. A composition comprising a cytochalasin and an antineoplastic agent.

10. A pharmaceutical composition comprising the composition of claim 9 and a pharmaceutically acceptable carrier or diluent.

11. The pharmaceutical composition of claim 9 wherein the antineoplastic agent is doxorubicin.

12. The pharmaceutical composition of claim 11 wherein the cytochalasin is cytochalasin B.

13. A method for inhibiting neoplastic cell growth in vitro comprising the step of administering synergistically-acting effective amounts of a synthetic or mold-derived membrane-acting compound and an antineoplastic agent to the cells.

14. The method of claim 13 wherein the synthetic or naturally-occurring membrane-acting compound is a cytochalasin.

15. The method of claim 14 wherein the cytochalasin is cytochalasin B.

16. The method of claim 15 wherein the antineoplastic agent is doxorubicin.

17. A synergistically-acting combination of a synthetic or mold-derived membrane-acting compound and an antineoplastic agent.

18. The synergistically-acting combination of claim 17 wherein the membrane-acting compound is a cytochalasin and the antineoplastic agent is doxorubicin.

19. A pharmaceutical composition comprising the synergistically-acting combination of claim 17 and a pharmaceutically acceptable carrier or diluent.

20. A composition comprising a mold-derived membrane-acting compound containing less than about 1% of other mold-derived membrane-acting compounds.

0297946

FIG. 1

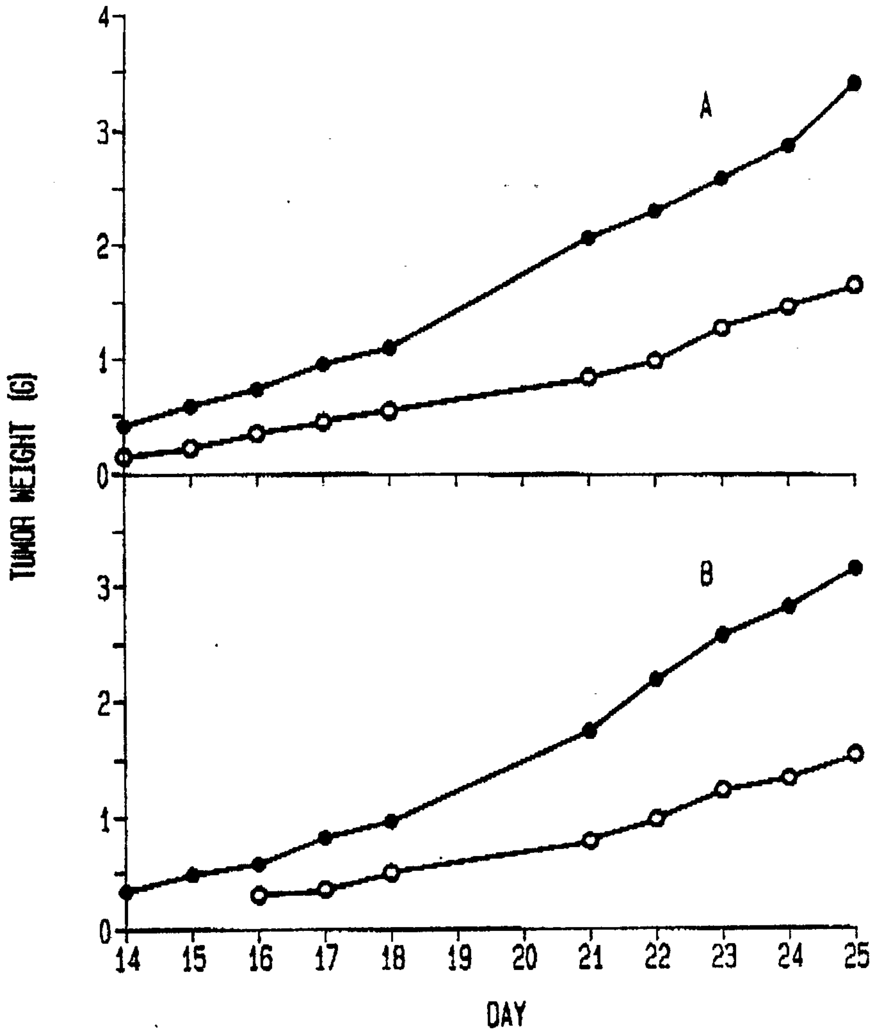


FIG. 2

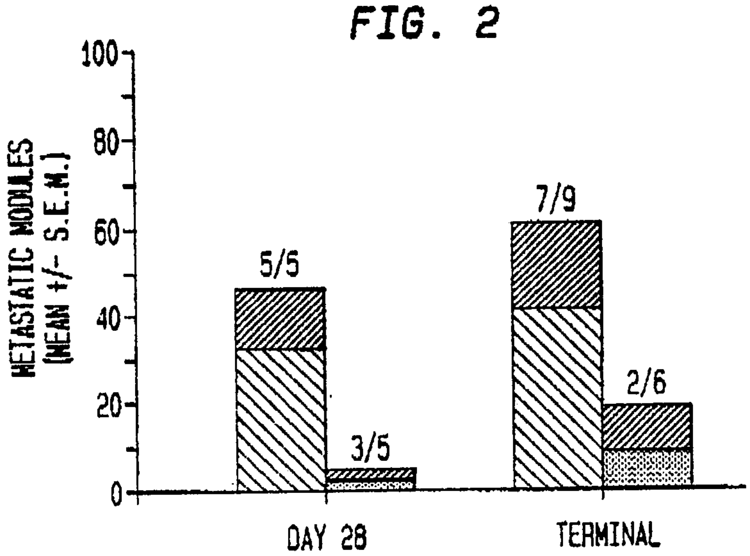

FIG. 3

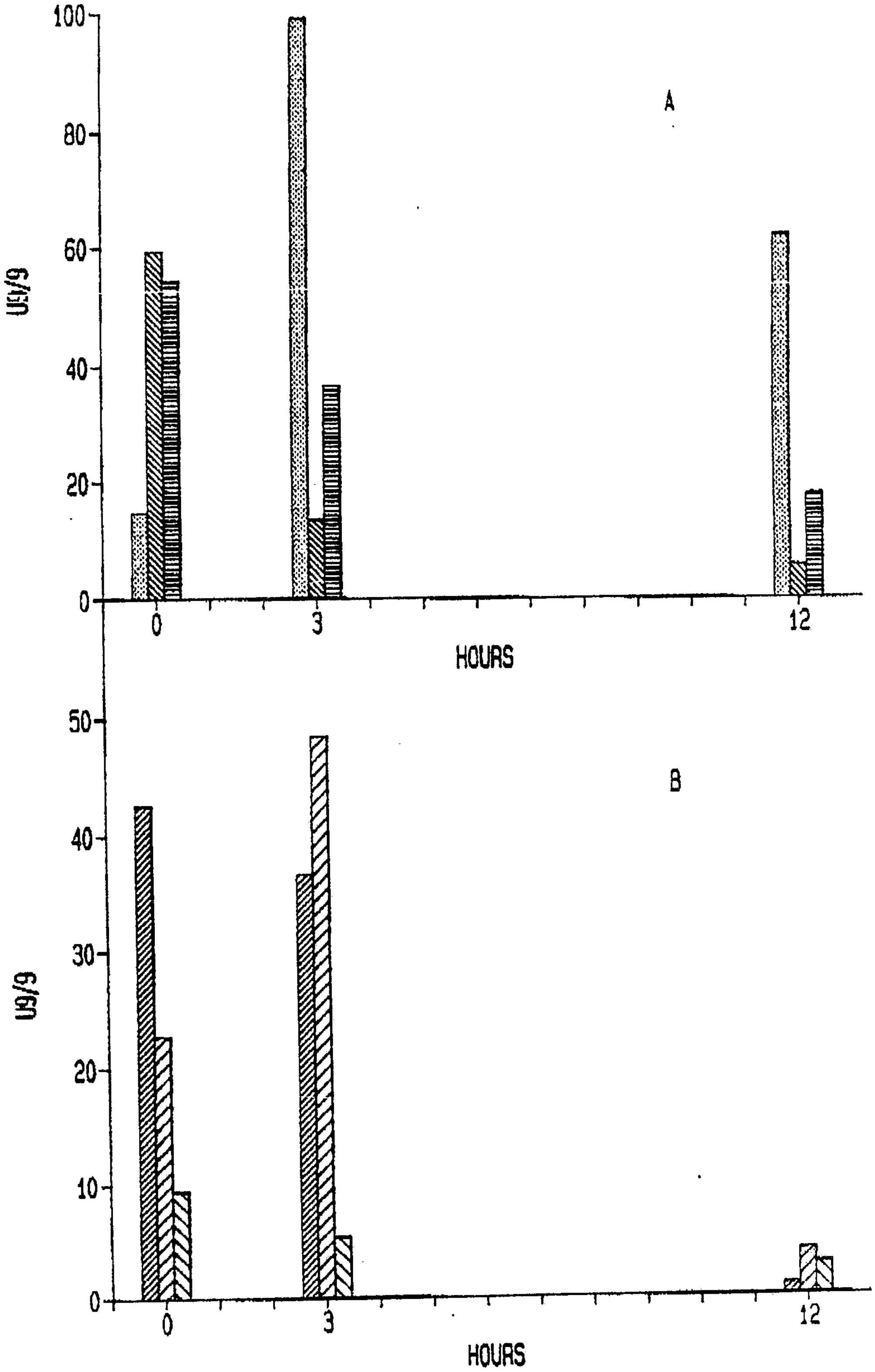

FIG. 4

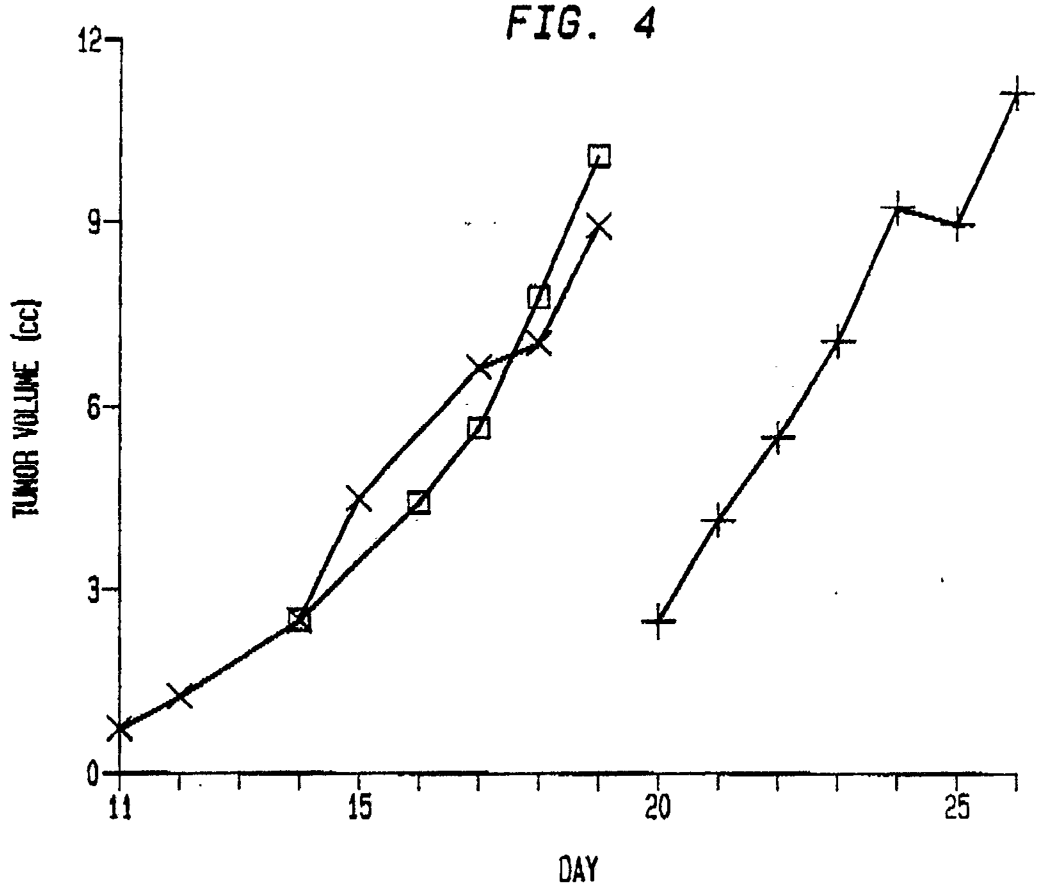
12
9
6
3
0
TUMOR VOLUME (cc)
11
15
20
25
DAY

FIG. 5

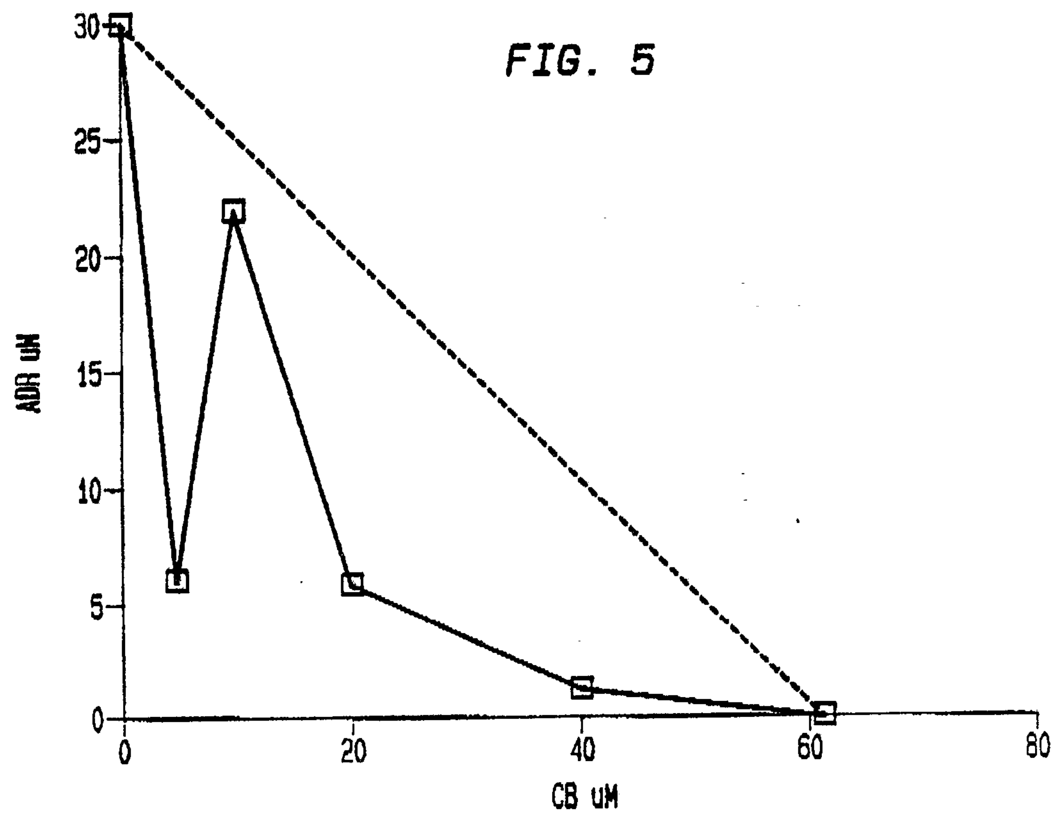
30
25
20
15
10
5
0
ADR uM
0
20
40
60
80
CB uM

FIG. 6

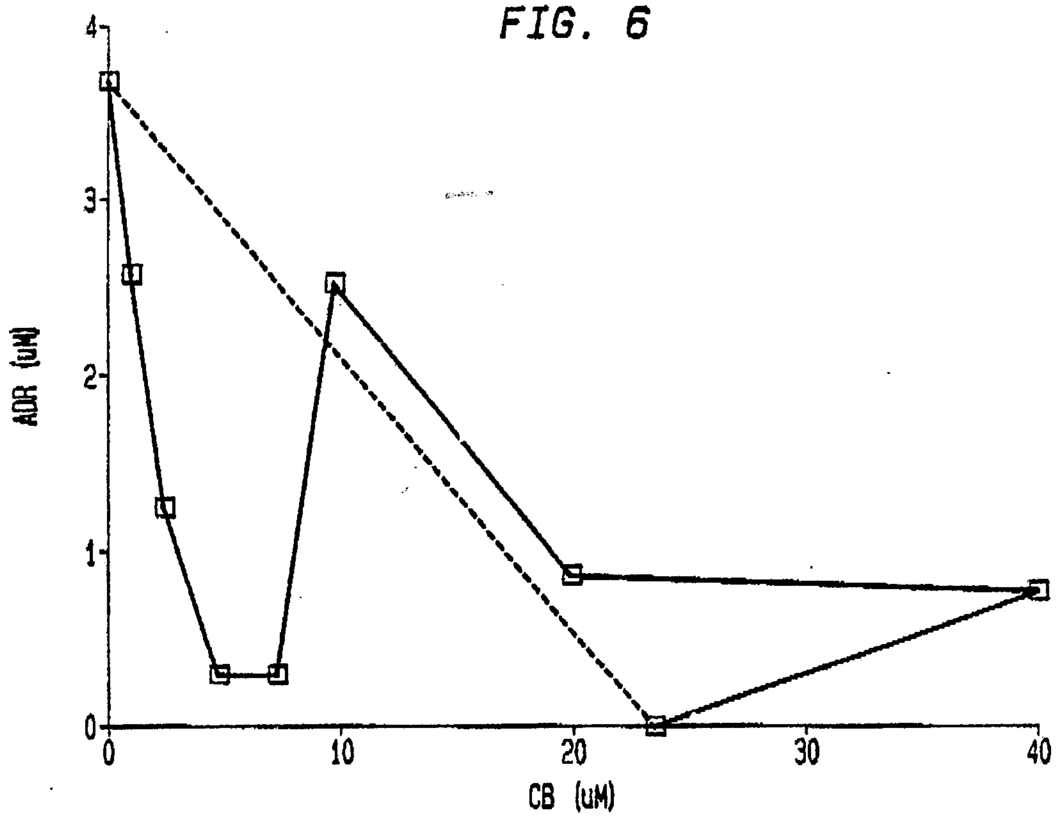


FIG. 7

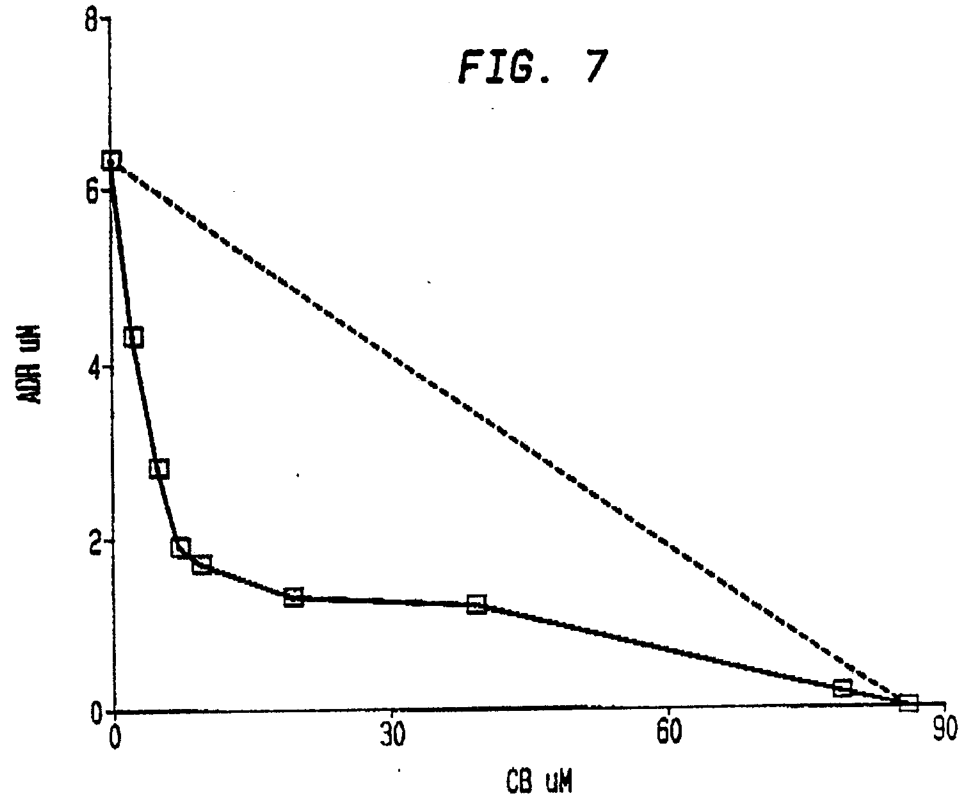

FIG. 8